# EUROPÄISCHE PATENTSCHRIFT

(11) **EP 0 445 644 B1**
(45) Veröffentlichungstag und Bekanntmachung des Hinweises auf die Patenterteilung: **24.05.1995**
(21) Anmeldenummer: 91102910.6
(22) Anmeldetag: 28.02.1991
(51) Int. Cl.: C07D 239/34, C07D 239/52, C07D 239/60, C07D 239/46

(54) **Trifluor- bzw. Chlordifluormethoxypyrimidine und Verfahren zu ihrer Herstellung**
Trifluoro- respectively chlorodifluoromethoxy pyrimidines and process for their preparation
Trifluoro- respectivement chlorodifluorométhoxy-pyrimidines et procédé pour leur préparation

(30) Priorität: 08.03.1990 DE 4007317
(43) Veröffentlichungstag der Anmeldung: 11.09.1991
(73) Patentinhaber: BASF Aktiengesellschaft, 67063 Ludwigshafen (DE)
(72) Erfinder: Hamprecht, Gerhard, Dr., W-6940 Weinheim (DE); Wolf, Hans-Josef, Dr., W-6701 Maxdorf (DE)

(56) Entgegenhaltungen:
- EP-A- 0 070 804
- DD-A- 234 865
- CHEMICAL ABSTRACTS, vol. 112, no. 19, 07 Mai 1990 Columbus, Ohio, USA Seite775; rechte Spalte; ref. no. 179016R & JP-A-01-238588 (ISHIHARA SANGYO KAISHA LTD) 22 September 1989

## Beschreibung

Die Vorliegende Erfindung betrifft neue substituierte Trifluor- bzw. Chlordifluormethoxy-pyrimidine der allgemeinen Formel I, 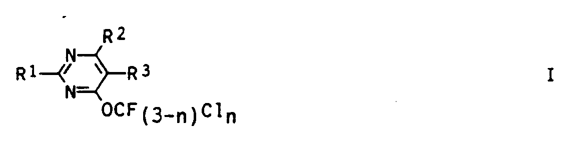
in der R¹ Fluor oder Chlor bedeutet und R² und R³ unabhängig voneinander für Wasserstoff, ein Halogenatom oder eine Halogenalkylgruppe, darüber hinaus R² auch für einen Trifluor- bzw. Chlordifluormethoxyrest steht, und n 0 oder 1 bedeuten und Verfahren zu ihrer Herstellung durch Umsetzung von 4-Trichlormethoxy-pyrimidinen mit Antimontrifluorid, gegebenenfalls in Gegenwart katalytischer Mengen eines Antimon(V)-salzes oder mit Fluorwasserstoff als Halogenaustauschmittel.

Weiterhin betrifft die vorliegende Erfindung die Verbindungen II und ein Verfahren zu deren Herstellung.

Die Verbindungen I und 11 dienen als Zwischenprodukte zur Herstellung organischer Verbindungen, insbesondere zur Herstellung von herbiziden Sulfonylharnstoffen.

Aufgrund der schwierigen Handhabung des äußerst reaktionsfähigen, unselektiven und giftigen Fluors sind bisher keine Methoden zur direkten Fluorierung von Alkoxy-pyrimidinen bekannt geworden. Trifluor- bzw. Chlordifluormethoxy-pyrimidine sind bisher nicht bekannt. Difluormethoxy-pyrimidine wurden auf umständlichem Weg, beispielsweise durch Etherspaltung von 2-Amino-4,6-dimethoxy-pyrimidin zur entsprechenden 4-Hydroxyverbindung und anschließende Umsetzung mit Chlordifluormethan zu dem 2-Amino-4-difluormethoxy-6-methoxy-pyrimidin in 10 % Ausbeute gewonnen (EP-A-70804, Beispiel 6). Da das Ausgangsmaterial auf Basis von Trichlorpyrimidin durch Reaktion mit Ammoniak und Methanol zuerst hergestellt, die Methylgruppe dann aber wieder abgespalten werden muß, ist dieser Zugang wenig wirtschaftlich. Die direkte Umsetzung Von 2-Amino-4,6-dihydroxy-pyrimidin mit Chlordifluormethan liefert die entsprechende 4,6-Bis(difluormethoxy)-verbindung in 6,4 % Ausbeute (EP 70804, Beispiel 3). Schließlich erfordert die Handhabung des schädlichen Chlordifluormethans besondere Sicherheitsvorkehrungen, um ein Entweichen in die Atmosphäre zu verhindern.

Der Erfindung lag nun die Aufgabe zugrunde, die erfindungsgemäßen Fluormethoxy-pyrimidine gegenüber dem Stand der Technik auf einfacherem Wege, in kürzerer Reaktionszeit in besseren Ausbeuten und ohne einen gleichzeitigen Austausch mehrerer Kernhalogenatome in hoher Selektivität herzustellen.

Demgemäß wurde gefunden, daß man die neuen Trifluor- bzw. Chlordifluormethoxy-pyrimidine der allgemeinen Formel I, 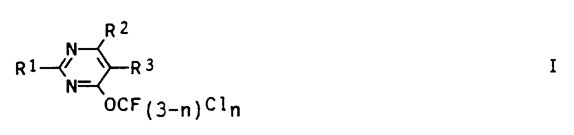
in der R¹, R² und R³ die vorgenannte Bedeutung besitzen, vorteilhaft erhält, wenn man Trichlormethoxypyrimidine der Formel II, 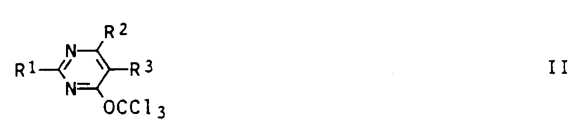
in der R¹ Fluor oder Chlor bedeutet und R² und R³ unabhängig voneinander für Wassertoff, ein Halogenatom oder eine C₁-C₄-Halogenalkylgruppe, darüber hinaus R² auch für eine Trichlormethoxygruppe steht, mit Antimontrifluorid, gegebenenfalls in Gegenwart katalytischer Mengen eines Antimon(V)-salzes oder mit Fluorwasserstoff als Halogenaustauschmittel behandelt.

Die Umsetzung kann für den Fall der Verwendung von 2,4-Dichlor-6-trichlormethoxy-pyrimidin und Antimontrifluorid bzw. Fluorwasserstoff durch folgendes Schema beschrieben werden: 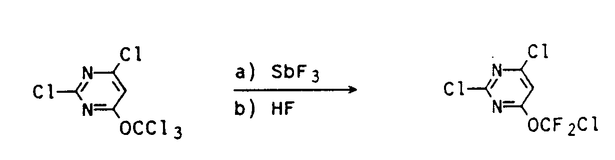
Im Fall der Verwendung von Antimontrifluorid und katalytischer Mengen Antimonpentachlorid bzw. Fluorwasserstoff läßt sich die Umsetzung durch folgendes Schema wiedergeben: 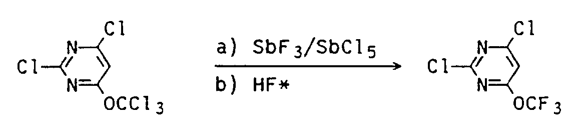
*) bei der Umsetzung mit HF können auch aromatisch gebundene Chloratome gegen Fluor ausgetauscht werden.

Im Fall der Verwendung von 2-Fluor-4-trichlormethyl-6-trichlormethoxy-pyrimidin läßt sich die Umsetzung durch folgendes Schema wiedergeben: 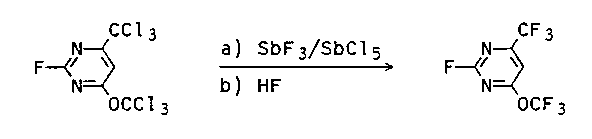
Das Verfahren liefert auf einfachem und wirtschaftlichem Weg neue Trifluormethoxy- bzw. Chlordifluormethoxy-pyrimidine in hoher Ausbeute und Reinheit. Zwei oder mehr anwesende, kernständige Chloratome werden hierbei nicht ausgetauscht. Im Hinblick auf den Stand der Technik sind alle diese vorteilhaften Eigenschaften überraschend.

Bevorzugte Endstoffe 1 und dementsprechend bevorzugte Ausgangsstoffe II sind solche, in deren Formeln R¹ Fluor oder Chlor bedeutet und R² und R³ unabhängig voneinander Wasserstoff, Fluor, Chlor, Brom, Trichlormethyl, Dichlorfluormethyl, Chlordifluormethyl, Trifluormethyl, 1,1-Dichlor-2,2,2-trifluorethyl, 1,1,2,2,2-Pentafluorethyl, 1,1,2,2,2-Pentachlorethyl bedeuten und darüber hinaus solche Endstoffe I, in denen R² zusätzlich für einen Trifluormethoxy- bzw. Chlordifluormethoxyrest steht, wenn in den entsprechenden Ausgangsstoffen 11 R² eine Trichlormethoxygruppe bedeutet und n für 0 oder 1 steht.

Zweckmäßig verwendet man einen überschuß Von 1 bis 200, vorzugsweise 5-20 Mol% Antimontrifluorid pro Trichlormethylequivalent. Die Katalysatormenge an Antimon(V)salz beträgt zwischen 1 bis 20, vorzugsweise 5-18 Mol% pro Trichlormethylequivalent. Vorzugsweise dosiert man den Ausgangsstoff II bei 90 bis 130°C zu der Mischung des Halogenaustauschmittels und erwärmt dann noch zwischen 10 und ca. 120 Minuten auf eine Temperatur zwischen 140 bis 170°C. Anschließend wird destillativ aufgearbeitet.

Man kann die Reaktion jedoch auch kontinuierlich führen, den Ausgangsstoff II bei 140 bis 170°C innerhalb 10 bis ca. 120 Minuten zugeben und gleichzeitig unter vermindertem Druck den entstehenden niedriger siedenden Endstoff I abdestillieren. Spuren mitgerissener Antimonsalze lassen sich durch Extraktion mit konz. Salzsäure beseitigen.

Arbeitet man ohne Antimon(V)salz-Katalyse oder setzt nur geringe Mengen, z.B. 0,2 bis 1 Mol% ein, und reduziert die Menge an Antimontrifluorid auf 60 bis 90 Mol% pro Trichlormethylequivalent, so bleibt der Halogenaustausch auf der Chlordifluormethoxystufe stehen.

Anstelle von Antimontrifluorid kann der Halogenaustausch auch mit Fluorwasserstoff bei 0 bis 170°C, vorzugsweise 40 bis 120°C, durchgeführt werden. Hierzu versetzt man in einem Autoklaven den Ausgangsstoff II mit einem überschuß Von 300 bis 700, vorzugsweise 350 bis 400 Mol% Fluorwasserstoff pro Trichlormethylequivalent und rührt 10 Minuten bis ca. 10 Stunden nach. Im allgemeinen ist eine Nachreaktionszeit von bis zu 4 Stunden ausreichend. Nach dem Entspannen und der Entfernung flüchtiger Bestandteile wird wie beschrieben aufgearbeitet.

Bevorzugte Endstoffe der Formel I im Hinblick auf deren Weiterverarbeitung zu herbiziden Sulfonylharnstoffderivaten sind beispielsweise 2,4-Difluor-6-trifluormethoxy-pyrimidin, 6-Chlordifluormethoxy-2,4-difluor-pyrimidin, 2,4-Dichlor-6-trifluormethoxy-pyrimidin, 6-Chlordifluormethoxy-2,4-dichlor-pyrimidin, 2-Chlor-4,6bis-trifluormethoxy-pyrimidin, 2-Chlor-4,6-bis-chlordifluormethoxy-pyrimidin, 2-Chlor-4-trifluormethoxy-6-trifluormethyl-pyrimidin, 2-Fluor-4-trifluormethoxy-6-trifluormethyl-pyrimidin, 4-Chlordifluormethyl-2-fluor-6-trifluormethyl-pyrimidin, 2,5-Dichlor-4-trifluormethoxy-pyrimidin, 4,6-Bis-trifluormethoxy-2,5-dichlor-pyrimidin, 2,5-Difluor-4-trifluormethoxy-pyrimidin, 4,6-Bis-trifluormethoxy-2,5-difluor-pyrimidin und 2-Fluor-4-trifluormethoxy-5-trifluormethyl-pyrimidin

Die für die Herstellung der fluorierten Pyrimidine I benötigten Trichlormethoxy-pyrimidine der Formel II 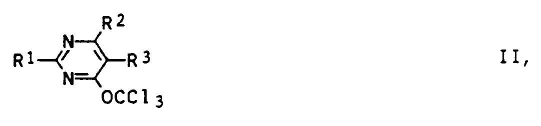
in der R¹, R² und R³ die eingangs erwähnte Bedeutung haben, erhält man vorteilhaft in der Weise, daß man Methoxy-pyrimidine der Formel III, 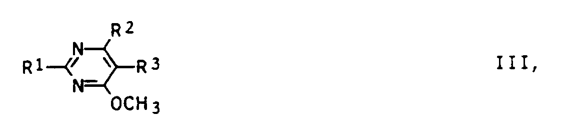
in der R¹ Fluor oder Chlor bedeutet und R² und R³ unabhängig voneinander Wasserstoff, ein Halogenatom oder eine C₁-C₄-Halogenalkylgruppe bedeuten und R² darüber hinaus auch für eine Methoxygruppe steht, mit einem Chlorierungsmittel behandelt.

Der Erfolg des Verfahrens ist überraschend, da man im Hinblick auf die gute Elektronendonorfähigkeit der Methoxygruppe in noch weitergehendem Maß Kernhalogenierung und Nebenreaktionen erwartet hatte. Eine ausgeprägte Tendenz zur Halogenierung in der 5-Position geht auch aus der Chlorierung des 6-Chloruracils in Wasser bei 80 °C zu dem 4,5-Dichlor-2,6-dihydroxy-pyrimidin in 60 % Ausbeute hervor (Isr. J. Chem. 1968, 6, 603).

Die Umsetzung kann für den Fall der Verwendung von 2,4-Dichlor-6-methoxy-pyrimidin und Chlor als Chlorierungsmittel durch folgendes Schema beschrieben werden. 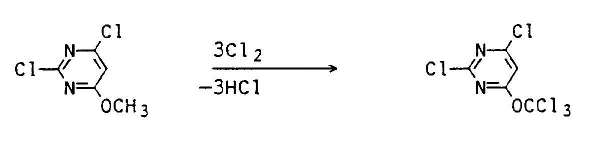
Das Verfahren liefert auf einfachem und wirtschaftlichem Weg neue Trichlormethoxy-pyrimidine in hoher Ausbeute und Reinheit ohne gleichzeitige Bildung von in 5-Stellung chlorierten Nebenprodukten. Im Hinblick auf den Stand der Technik sind alle diese vorteilhaften Eigenschaften überraschend.

Bevorzugte Zwischenprodukte II und dementsprechend bevorzugte Ausgangsstoffe III sind solche, in deren Formeln R¹ Fluor oder Chlor und R² und R³ unabhängig voneinander Wasserstoff, Fluor, Chlor, Brom, Trichlormethyl, Dichlorfluormethyl, Chlordifluormethyl, Trifluormethyl, 1,1-Dichlor-2,2,2-trifluorethyl, 1,1,2,2,2-Pentafluorethyl, 1,1,2,2,2-Pentachlorethyl bedeuten und darüber hinaus solche Produkte II, in denen R² zusätzlich für einen Trichlormethoxyrest steht, wenn in den entsprechenden Ausgangsstoffen R² eine Methoxygruppe bedeuten.

Als Chlorierungsmittel sind elementares Chlor oder Chlor abgebende Substanzen wie Sulfurylchlorid oder Phosphorpentachlorid geeignet. Chlor kann dabei auch in situ durch Oxydation von Chlorwasserstoffsäure, beispielsweise mit Braunstein oder durch anodische Chlorierung hergestellt werden.

Die Umsetzung kann in Gegenwart eines inerten Lösungsmittels, beispielsweise einem chlorierten Kohlenwasserstoff wie Chloroform, Tetrachlorkohlenstoff, Chlorbenzol, 1,2-, 1,3- oder 1,4-Dichlorbenzol, einem Nitril wie Acetonitril, Propionitril, einer Nitroverbindung wie Nitrobenzol, einer Carbonsäure wie Essigsäure, Propionsäure, einem Säureanhydrid wie Essigsäureanhydrid, einem Säurechlorid wie Chloracetylchlorid, α-Chlorpropionsäurechlorid, α,α-Dichlorpropionsäurechlorid, einem anorganischen Säurehalogenid wie Phosphortrichlorid oder Phosphoroxychlorid oder bevorzugt ohne Lösungsmittel in der Schmelze des Ausgangsstoffes III durchgeführt werden.

Gegebenenfalls kann man die Reaktion durch Zugabe eines Radikalstarters beschleunigen; als solcher eignet sich die Bestrahlung mit Licht, vorzugsweise UV-Licht oder die Zugabe von α,α'-Azoisobutyronitril in zweckmäßig einer Menge von 0,2 bis 7 Mol%, bezogen auf den Ausgangsstoff III. Man kann die Reaktion auch durch Zugabe eines Katalysators beschleunigen; als solcher eignet sich Phosphorpentachlorid in zweckmäßig einer Menge von 0,5 bis 7 Mol% bezogen auf den Ausgangsstoff III. In diesem Fall legt man den Ausgangsstoff III zusammen mit dem Katalysator vor und beginnt dann mit der Chlorierung. Statt des Phosphorpentachlorids kann man auch eine dieses unter den Reaktionsbedingungen bildende Ausgangskomponente, z.B. Phosphortrichlorid oder gelben Phosphor, zugeben und dann mit der Chlorierung beginnen.

Ausgangsstoff III kann mit Chlor in annähernd stöchiometrischer Menge oder vorzugsweise im überschuß, vorteilhaft mit 3,1 bis 11, insbesondere 3,3 bis 5 Mol Chlor je Equivalent Methoxy in dem Ausgangsstoff III umgesetzt werden. Die Umsetzung wird bei einer Temperatur von 60 bis 180°C, vorteilhaft von 100 bis 150 °C, drucklos oder unter Druck, kontinuierlich oder diskontinuierlich durchgefürt.

Chloriert man bei 1 bar, so werden zweckmäßig 3,3 bis 5 Mol Chlorgas, bezogen auf ein Equivalent Methoxy in dem Ausgangsstoff III, eingesetzt, was einem Chlorumsatz von 91 bis 60 % entspricht. Durch geeignete apparative Maßnahmen, z. B. durch Anwendung von mäßigem Überdruck, zweckmäßig 1 bis 10 bar, oder durch Verwendung einer Blasensäule, läßt sich der Chlorumsatz erhöhen. Vorteilhaft läßt man das Chlorgas möglichst lange mit der organischen Phase in Berührung kommen, indem diese beispielsweise stark gerührt wird oder das Chlorgas eine dicke Schicht der organischen Phase durchdringen muß.

Die Reaktionszeit beträgt im allgemeinen etwa 0,5 bis 12 Stunden.

In einer bevorzugten Ausführungsform des Verfahrens geht man so vor, daß man innerhalb von 0,5 bis 12 Stunden, Vorzugsweise 1 bis 10 Stunden die erforderliche Menge Chlorgas unter intensivem Rühren in den flüssigen Ausgangsstoff III einleitet, wobei man zunächst bei einer Temperatur von 60 bis 80°C beginnt und diese - gegebenenfalls unter Ausnutzung des exothermen Charakters der Reaktion - kontinuierlich steigert, so daß gegen Ende die Umsetzung bei einer Temperatur von 100 bis 150°C durchgeführt wird. Es ist selbstverständlich, daß bei größeren Reaktionsansätzen dem exothermen Charakter durch äußere Kühlung bsw. geeignete Dosierung der Chlormenge Rechnung getragen werden muß; mit dem Abflauen der Reaktion entfernt man das Kältebad und kann gegebenenfalls noch nacherhitzen.

Die Aufarbeitung und Isolierung der Endstoffe kann in üblicher Weise erfolgen. Beispielsweise kann man aus der heißen organischen Phase mittels eines Inertgases Reste an Chlorwasserstoff, Chlor oder Katalysator austragen; dabei hinterbleibt in hoher Ausbeute ein bereits recht reines Rohprodukt. Durch Destillation oder Chromatographie kann es weiter gereinigt oder aber direkt für weitere Umsetzungen eingesetzt werden.

Bevorzugte Verbindungen der Formel II sind beispielsweise 2-, 4-Trichlomethoxy-pyrimidin, 2,4-Difluor-6-trichlormethoxy-pyrimidin, 2-Fluor-4,6-bis-trichlormethoxy-pyrimidin, 2, 4-Dichlor-6-trichlormethoxy-pyrimidin, 2-Chlor-4,6-bis-trichlormethoxy-pyrimidin, 2-Chlor-4-trichlormethoxy-6-trichlormethyl-pyrimidin, 2-Chlor-4-trichlormethoxy-6-trifluormethylpyrimidin, 2-Fluor-4-trichlormethoxy-6-trichlormethyl-pyrimidin, 2-Fluor-4-trichlormethoxy-6-trifluormethyl-pyrimidin, 2,5-Dichlor-4-trichlor-methoxy-pyrimidin, 4,6-Bis-trichlormethoxy-2,5-dichlorpyrimidin, 2,5-Difluor-4-trichlormethoxy-pyrimidin, 4,6-Bis-trichlormethoxy-2,5-difluorpyrimidin, 2-Chlor-4-trichlormethoxy-5-trifluormethyl-pyrimidin, 2-Fluor-4-trichlormethoxy-5-trifluormethyl-pyrimidin, 2-Chlor-4-trichlormethoxy-6-trifluormethyl-pyrimidin und 2-Fluor-4-trichlormethoxy-6-trifluormethyl-pyrimidin.

Die neuen Trichlormethoxy-pyrimidine II sowie die neuen Trifluor- bzw. Chlordifluormethoxypyrimidine I sind wertvolle Zwischenprodukte für die Herstellung von Pharmazeutika, Farbstoffen und Pflanzenschutzmitteln.

Beispielsweise können die Verbindungen I z.B. 2,4-Dichlor- oder 2,4-Difluor-6-trifluormethoxypyrimidin, mit Ammoniak und Methanol in das entsprechende 2-Amino-6-methoxy-4-trifluormethoxy-pyrimidin umngewandelt werden, das mit 2-Carbomethoxy-benzolsulfonylisocyanat zu herbiziden Sulfonylharnstoffen reagiert.

Solche Folgeumsetzungen sind in den zeitgleichen deutschen Anmeldungen P 40 07 316.5 und P 40 07 683.0 beschrieben.

### Beispiele

### Beispiel 1

### I Herstellbeispiele für die Vorprodukte

### Beispiel I.1

### 2-Chlor-4-trichlormethoxy-6-trichlormethylpyrimidin

a) 2-Chlor-4-methoxy-6-trichlormethylpyrimidin
   293,1 g (1,692 mol) 30 %ige Natriummethylatlösung wurden innerhalb 1 1/2 Stunden bei 0 bis 5°C unter Rühren zu einer Lösung von 434 g (1,692 mol) 2,6-Dichlor-4-trichlormethylpyrimidin in 1 1 1,2-Dichlorethan gegeben. Es wurde 1 Stunde bei 0 bis 5°C und 12 Stunden bei 25°C gerührt. Das Reaktionsgemisch wurde 4x mit Wasser und 3x mit gesättigter Kochsalzlösung extrahiert. Nach dem Trocknen über Magnesiumsulfat und Einengen erhielt man 423 g (95 % d. Th.) der Titelverbindung als fast farbloses öl. ¹H-NMR (CDCl₃) (ppm) OCH₃ (s/3H) 4,1; CH (s/1H) 7,25.
b) 2-Chlor-4-trichlormethoxy-6-trichlormethylpyrimidin
   In eine Mischung von 210 g (0,802 mol) 2-Chlor-4-methoxy-6-trichlormethylpyrimidin und 260 mg (0,0016 mol), α,α'-Azoisobutyronitril wurden unter UV-Bestrahlung und gaschromatographischer Kontrolle des Reaktionsverlaufs bei einer Temperatur von zunächst 110°C Chlor eingeleitet, wobei sich auch nach Entfernen der Heizbades eine Reaktionstemperatur von 140°C einstellte. Nach dem Abklingen der Reaktion wurden während 5 1/2 Stunden bei 120°C insgesamt 341 g (4,8 mol) Chlor eingeleitet. Zu dem erkaltenden Reaktionsgemisch rührte man ab 40°C 70 ml n-Pentan zur Ausfällung hinzu. Der Niederschlag wurde abgesaugt, mit Petrolether gewaschen und getrocknet, wobei man 163 g (55 % d. Th.) der Titelverbindung vom Fp. 67-69°C erhielt.
   Das Filtrat (113,8 g) bestand nach dem Gaschromatogramm zu 83 % aus der Titelverbindung, 4 % 2-Chlor-4-dichlormethoxy-6-trichlormethyl-pyrimidin und 9 % 2,4-Dichlor-6-trichlormethylpyrimidin. Die Gesamtausbeute der Titelverbindung betrug 87,6 % der Theorie.

### Beispiel I.2

### 2,4-Difluor-6-trichlormethoxy-pyrimidin

a) 2,4-Difluor-6-methoxypyrimidin
   (Herstellung nach dem verfahren der älteren deutschen Patentanmeldung P 39 00 471.6 vom 10.01.89 (O.Z. 0050/40474)
   Zu einer Mischung aus 250 g (1,865 mol) 2, 4, 6-Trifluorpyrimidin in 1,4 l Methanol wurden bei -20°C innerhalb von 45 Minuten 335,8 g (1,865 mol) 30 %iges Natriummethylat (in Methanol) gegeben und weitere 30 Minuten bei dieser Temperatur gerührt. Anschließend ließ man auf 25 °C erwärmen und engte die Reaktionsmischung auf ca. 1/5 ihres Volumens ein.
   Das so erhaltene Gemisch wurde zwischen Diethylether und Wasser verteilt, wonach die organische Phase über Magnesiumsulfat getrocknet und eingeengt wurde. Nach Destillation erhielt man 141,6 g (52 % d.Th.) der Titelverbindung von Kp.: 144-145 °C.
   Aus dem Destillationsrückstand erhielt man durch Destillation über einen Normag-Aufsatz 114,4 g (42 % d. Th.) an 4,6-Difluor-2-methoxy-pyrimidin vom Kp.: 157-161°C.
b) 2,4-Difluor-6-trichlormethoxypyrimidin
   210 g (2,96 mol) Chlor wurden unter UV-Bestrahlung und gaschromatographischer Kontrolle des Reaktionsverlaufs während 2 1/2 Stunden unter Rühren bei 130°C in 123 g (0,843 mol) 2,4-Difluor-6-methoxy-pyrimidin eingeleitet. Das Reaktionsgemisch wurde über eine 10-cm-Vigreux-Kolonne im Vakuum destilliert, wobei man 190,2 g (90,5 % d. Th.) der Titelverbindung vom Kp. 40-43°C/0,2 mbar erhielt.

### Beispiel I.3

### 2, 4-Dichlor-6-trichlormethoxy-pyrimidin

Unter Rühren, UV-Bestrahlung und gaschromatographischer Kontrolle des Reaktionsverlaufs wurden 303 g (4,27 mol) Chlor während 1/2 Stunde bei 80°C, 1 Stunde bei 100°C, 3 Stunden bei 120°C und 3 Stunden bei 150°C in eine Mischung von 209 g (1,168 mol) 2,6-Dichlor-4-methoxy-pyrimidin und 2 g (0,012 mol) α,α'-Azoisobutyronitril eingeleitet. Anschließend wurde das Reaktionsgemisch im Vakuum destilliert. Man erhielt 241,3 g (73 % d. Th.) der Titelverbindung vom Kp. 87-88°C/0,4 mbar; Fp. 55-56°C.

### II Umsetzung zu den Endprodukten I

### Beispiel II.1

### 2, 4-Difluor-6-trifluormethoxy-pyrimidin

49,9 g (0,2 mol) 2,4-Difluor-6-trichlormethoxy-pyrimidin wurde bei 100°C innerhalb 15 Minuten unter Rühren zu einer Mischung von 39,3 g (0,22 mol) Antimontrifluorid und 9,38 g (0,031 mol) Antimonpentachlorid gegeben. Innerhalb 25 Minuten wurde die Badtemperatur von 100 auf 150°C erhöht und 30 Minuten nachgerührt, wobei sich zwischen 120 bis 125°C Rückfluß einstellte. Durch anschließende Destillation erhielt man 37,1 g (92,7 % d. Th.) der Titelverbindung vom Kp. 125-127°C.

### Beispiel II.2

### 6-Chlordifluormethoxy-2,4-difluor-pyrimidin

93 g (0,373 mol) 2,4-Difluor-6-trichlormethoxy-pyrimidin wurden bei 100°C innerhalb 10 Minuten unter Rühren zu einer Mischung von 44,5 g (0,249 mol) Antimontrifluorid und 0,94 g (0,0031 mol) Antimonpentachlorid gegeben. Innerhalb 25 Minuten wurde die Badtemperatur von 100 auf 175°C erhöht, wobei sich bei 145°C Rückfluß einstellte. Nach 1 1/2 Stunden Rühren wurde das Reaktionsprodukt bei 146-150°C abdestilliert. Das Destillat wurde in Methylenchlorid gelöst, mit 6 n Salzsäure extrahiert und über Magnesiumsulfat getrocknet. Nach dem Einengen im Vakuum erhielt man als Rückstand die Titelverbindung in einer Ausbeute von 63,7 g = 78,8 % d. Th.

### Beispiel II.3

### 2-Fluor-4-trifluormethoxy-6-trifluormethyl-pyrimidin

80 g (0,219 mol) 2-Chlor-4-trichlormethyl-6-trichlormethoxy-pyrimidin wurden innerhalb 5 Minuten unter Rühren bei 100° zu einer Mischung von 93,9 g (0,525 mol) Antimontrifluorid und 18,7 g (0,0627 mol) Antimonpentachlorid gegeben. Innerhalb 10 Minuten wurde die Badtemperatur auf 140°C erhöht und 1 Stunde nachgerührt, wobei sich starker Rückfluß einstellte. Das Reaktionsprodukt wurde bei 135-140°C, gegen Schluß bei 95°C/50 mbar, überdestilliert. Das Destillat wurde in Methylenchlorid aufgenommen, mit 6 n Salzsäure extrahiert und über Magnesiumsulfat getrocknet. Nach dem Einengen im Vakuum erhielt man die Titelverbindung in einer Ausbeute von 35,9 g (65,5 % d. Th.).

### Beispiel II.4

### 2,4-Dichlor-6-trifluormethoxy-pyrimidin

115 g (0,407 mol) 2,4-Dichlor-6-trichlormethoxy-pyrimidin wurden innerhalb 5 Minuten unter Rühren bei 100°C zu einer Mischung von 80 g (0,447 mol) Antimontrifluorid und 18,77 g (0,0627 mol) Antimonpentachlorid gegeben, wobei sich die Reaktionstemperatur bis auf 140°C erhöhte. Es wurde noch 45 Minuten bei 150°C gerührt. Zur Destillation wurde ein Druck von 210 mbar eingestellt, wobei die Titelverbindung bei 128°C überging; letzte flüchtige Bestandteile wurden bei 110°C/22 mbar übergetrieben. Das Destillat wurde in Methylenchlorid gelöst, mit 6 n Salzsäure extrahiert und über Magnesiumsulfat getrocknet. Nach dem Einengen im Vakuum erhielt man die Titelverbindung in einer Ausbeute von 80 g (84,4 % d. Th.) als farbloses öl vom n_{D}²⁵ = 1,4604.

### III Umsetzung der Verbindungen I zu herbizid wirksamen Sulfonylharnstoffderivaten

### Beispiel III.1

### 2-Amino-4-chlordifluormethoxy-6-fluor-pyrimidin

9,8 g (0,578 mol) gasförmiges Ammoniak wurden innerhalb einer Stunde bei -75 bis -70°C unter Rühren in eine Mischung von 62,5 g (0,289 mol) 2,4-Difluor-6-chlordifluormethoxy-pyrimidin in 300 ml Tetrahydrofuran eingeleitet. Es wurde eine Stunde bei -70°C gerührt und dann auf Raumtemperatur erwärmt. Der ausgefallene Niederschlag wurde abgesaugt, zwischen Essigester und Wasser verteilt und die organische Phase über Magnesiumsulfat getrocknet. Das Reaktionsfiltrat wurde eingeengt, in der obigen Essigesterphase gelöst, über Kieselgel mit Petrolether:Ether = 5:1 chromatographiert und eingeengt. Man erhielt 46,5 g (75,3 % d. Th.) der Titelverbindung als farblose Kristalle vom Fp. 77-80°C.

### Beispiel III.2

### 2-Amino-4-fluor-6-trifluormethoxy-pyrimidin

8,7 g (0,51 mol) gasförmiges Ammoniak wurden innerhalb 1 Stunde bei -75 bis -70°C unter Rühren in eine Mischung von 51 g (0,255 mol) 2,4-Difluor-6-trifluormethoxy-pyrimidin in 200 ml Diethylether eingeleitet. Es wurde noch 1 1/2 Stunden bei -70°C und 1 Stunde bei Raumtemperatur gerührt. Das Reaktionsgemisch wurde im Vakuum eingeengt, in Methylenchlorid aufgenommen und mit Wasser extrahiert. Nach dem Trocknen der organischen Phase, Einengen und Chromatographieren über Kieselgel mit Petrolether:Ether = 8:1 erhielt man 38,1 g (75,6 % d. Th.) der Titelverbindung als farblose Kristalle vom Fp. 86-89°C.

### Beispiel III.3

### 2-Amino-4-chlor-6-trifluormethoxy-pyrimidin

4,3 g (0,25 mol) gasförmiges Ammoniak wurden innerhalb 45 Minuten unter Rühren bei -50 bis -45°C in eine Mischung von 23,3 g (0,1 mol) 2,4-Dichlor-6-trifluormethoxy-pyrimidin in 150 ml Methyl-tert.-butylether eingeleitet. Es wurde 30 Minuten bei -50°C, 1 Stunde bei -30°C und 1 Stunde bei 25°C gerührt. Der ausgefallene Niederschlag wurde abgesaugt, mit Wasser gewaschen und getrocknet, wobei man 5,4 g (33,1 % d. Th.) 4-Amino-2,4-dichlorpyrimidin vom Fp. 270-272°C als Nebenprodukt erhielt. Das Filtrat wurde mit Wasser gewaschen, getrocknet, teilweise im Vakuum eingeengt und mit Petrolether:Ether = 5:1 frakioniert chromatographiert, wobei man in den ersten Fraktionen 3 g (12,8 % d. Th.) Ausgangsmaterial als farbloses Öl und im Nachlauf 9 g (42 % d. Th.) der Titelverbindung als farblose Kristalle vom Fp. 55-56°C erhielt. Der Umsatz betrug 48,3 %.

### Beispiel III.4

### 4-Chlordifluormethoxy-6-fluor-2-methylamino-pyrimidin

20,3 g (0,0938 mol) 4-Chlordifluormethoxy-2,6-difluor-pyrimidin wurden in 150 ml Tetrahydrofuran vorgelegt und bei -70 bis -60°C innerhalb 30 Minuten unter Rühren mit 5,8 g (0,188 mol) gasförmigem Methylamin versetzt. Es wurde jeweils 1 Stunde bei -70°C, 0°C und 25°C gerührt. Nach dem Einengen des Reaktionsgemisches im vakuum wurde der Rückstand mit Wasser verrührt, 2x mit Essigester extrahiert und der Extrakt über Magnesiumsulfat getrocknet. Es wurde zum Teil im Vakuum eingeengt und dann über Kieselgel mit Ether/Petrolether 1:5 fraktioniert chromatographiert. Die ersten Fraktionen enthielten die Titelverbindung vom Fp. 57-61 °C in einer Ausbeute von 12,5 g (58,5 %).

### Beispiel III.5

### 2-Amino-4-trifluormethoxy-6-trifluormethyl-pyrimidin

4,7 g (0,278 mol) gasförmiges Ammoniak wurden unter Rühren bei -75 bis -70°C innerhalb 1 Stunde in eine Mischung von 38,0 g (0,147 mol) 2-Fluor(chlor)-4-trifluormethoxy-6-trifluormethyl-pyrimidin in 150 ml Diethylether eingeleitet. Es wurde jeweils 2 Stunden bei -75°C und nach dem Erwärmen bei 25°C gerührt. Nach dem Absaugen von dem ausgefallenen Niederschlag wurde die organische Phase mit Wasser extrahiert, getrocknet und teilweise eingeengt. Nach dem Chromatographieren mit Methyl-tert.-butylether über Kieselgel erhielt man 20,4 g (56,1 % d. Th.) der Titelverbindung vom Fp. 47-49°C.

### Beispiel III.6

### 2-Amino-4-methoxy-6-trifluormethoxy-pyrimidin

2,7 g (0,015 mol) 30%iges Natriummethylat wurden innerhalb 15 Minuten unter Rühren bei -5 bis 0°C Zu 2,95 g (0,015 mol) 2-Amino-4-fluor-6-trifluormethoxy-pyrimidin in 50 ml Methanol gegeben. Nach 1 Stunde Rühren bei 0°C und Erwärmen auf 25°C wurde das Reaktionsgemisch im Vakuum eingeengt, mit Wasser verrührt und 2x mit Methylenchlorid extrahiert. Nach dem Trocknen und Einengen im vakuum erhielt man 3,1 g (98 % d. Th.) der Titelverbindung mit n_{D}²⁵ = 1,4770.

### Beispiel III.7

### 2-Amino-4-chlordifluormethoxy-6-methoxy-pyrimidin

26,1 g (0,145 mol) 30%iges Natriummethylat wurden innerhalb 15 Minuten unter Rühren bei -10 bis 0°C zu 31,0 g (0,145 mol) 2-Amino-4-chlordifluormethoxy-6-fluorpyrimidin in 300 ml Methanol gegeben. Es wurde 30 Minuten bei 0°C und 1 Stunde bei 25°C gerührt. Das Reaktionsgemisch wurde im Vakuum eingeengt und wie oben aufgearbeitet. Man erhielt 31,6 g (96,6 % d. Th.) der Titelverbindung als farbloses Öl mit n_{D}²² = 1,5039.

### Beispiel III.8

### 4-Chlordifluormethoxy-2-methylamino-6-methoxy-pyrimidin

4,7 g (0,026 mol) 30%iges Natriummethylat wurden innerhalb 10 Minuten unter Rühren bei 0°C zu 6,0 g (0,0263 mol) 4-Chlordifluormethoxy-6-fluor-2-methylamino-pyrimidin in 100 ml Methanol gegeben. Es wurde jeweils 1 Stunde bei 0°C und bei 25°C gerührt. Nach üblicher Aufarbeitung erhielt man 6,3 g (100 % d. Th.) der Titelverbindung vom Fp. 49-53°C.

### Beispiel III.9

### 4-Chlordifluormethoxy-6-dimethylamino-2-methylamino-pyrimidin

1,9 g (0,0417 mol) gasförmiges Dimethylamin wurden innerhalb 10 Minuten unter Rühren bei 0°C in eine Mischung von 8,9 g (0,0417 mol) 2-Amino-4-chlordifluormethoxy-6-fluor-pyrimidin in 100 ml Tetrahydrofuran eingeleitet. Es wurde 1 Stunde bei 0°C und 2 Stunden bei 25°C gerührt. Nach üblicher Aufarbeitung erhielt man 9,7 g (97,5 % d. Th.) der Titelverbindung vom Fp. 127-130°C.

### Beispiel III.10

### 2-(((4-Fluor-6-trifluormethoxy-1, 3-pyrimidin-2-yl)aminocarbonyl)-aminosulfonyl)-benzoesäuremethylester

3,6 g (0,015 mol) 2-Isocyanatosulfonyl-benzoesäuremethylester in 15 ml 1,2-Dichlorethan wurden innerhalb 15 Minuten bei 25°C unter Rühren zu einer Mischung von 2,95 g (0,015 mol) 2-Amino-4-fluor-6-trifluormethoxy-pyrimidin in 100 ml 1,2-Dichlorethan gegeben und 12 Stunden bei 25°C gerührt. Die Reaktionslösung wurde im Vakuum eingeengt und der Rückstand mit Ether/Petrolether 1:1 verrührt. Nach dem Absaugen und Trocknen erhielt man 4,8 g (73,3 % d. Th.) der Titelverbindung vom Fp. 157-161°C.

### Beispiel III.11

### 2-(((4-Chlor-6-trifluormethoxy-1,3-pyrimidin-2-yl)aminocarbonyl)-aminosulfonyl)-benzoesäure-ethylester

2,55 g (0,01 mol) 2-Isocyanatosulfonyl-benzoesäure-ethylester in 10 ml Methylenchlorid wurden innerhalb 10 Minuten unter Rühren bei 25°C zu einer Mischung von 2,1 g (0,01 mol) 2-Amino-4-chlor-6-trifluormethoxy-pyrimidin in 100 ml Methylenchlorid gegeben. Es wurde 12 Stunden bei 25°C gerührt und von wenig Unlöslichem abgesaugt. Nach dem Einengen des Filtrats im Vakuum, verrühren des Rückstandes mit Ether/Petrolether 1:1, Absaugen und Trocknen erhielt man 4,0 g (85,4 % d. Th.) der Titelverbindung vom Fp. 148-151°C.

### Beispiel III.12

### 2-(((4-Methoxy-6-trifluormethoxy-1, 3-pyrimidin-2-yl)aminocarbonyl)-aminosulfonyl)-benzoesäuremethylester

4,8 g (0,02 mol) 2-Isocyanatosulfonyl-benzoesäuremethylester in 10 ml Acetonitril wurden innerhalb 15 Minuten unter Rühren bei 25°C zu einer Mischung von 4,1 g (0,02 mol) 2-Amino-4-methoxy-6-trifluormethoxy-pyrimidin in 100 ml Acetonitril gegeben und 12 Stunden nachgerührt. Nach dem Abtrennen des ausgefallenen Niederschlages (2,4 g vom Fp. 141-143°C) wurde das Filtrat im Vakuum eingeengt und mit Ether/Petrolether verrührt, abgesaugt und getrocknet. Man erhielt weitere 4,3 g der Titelverbindung vom Fp. 141-143°C. Die Gesamtausbeute betrug 6,7 g (74,4 % d. Th.).

### Beispiel III.13

### 2-(((4-Methoxy-6-trifluormethoxy-1,3-pyrimidin-2-yl)aminocarbonyl)-aminosulfonyl)-benzoesäuremethylester-natriumsalz

2,4 g (0,053 mol) 2-(((4-Methoxy-6-trifluormethoxy-1,3-pyrimidin-2-yl)-aminocarbonyl)aminosulfonyl)-benzoesäuremethylester wurden in 50 ml Methanol gelöst, bei 25°C mit 1,0 g (0,053 mol) 30%iger Natriummethylatlösung in Methanol versetzt und 10 Minuten gerührt. Nach dem Abdestillieren des Lösungsmittels bei vermindertem Druck erhielt man 2,5 g (100 % d.Th.) der Titelverbindung vom Fp. 175°C.

## Patentansprüche

1. Substituierte Trifluor- bzw. Chlordifluormethoxy-pyrimidine der allgemeinen Formel I 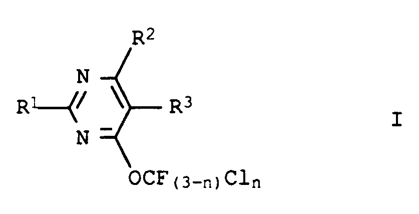 in der R¹ Fluor oder Chlor bedeutet und R² und R³ unabhängig voneinander für Wasserstoff, ein Halogenatom oder eine C₁-C₄-Halogenalkylgruppe, darüber hinaus R² auch für einen Trifluor- bzw. Chlordifluormethoxyrest steht und n 0 oder 1 bedeutet.

2. Verfahren zur Herstellung von Trifluor- bzw. Chlordifluormethoxy-pyrimidinen der allgemeinen Formel I nach Anspruch 1, dadurch gekennzeichnet, daß man Trichlormethoxy-pyrimidinverbindungen der Formel II 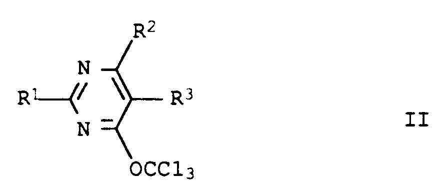 in der R¹ Fluor oder Chor bedeutet und R² und R³ unabhängig voneinander Wasserstoff, ein Halogenatom oder eine C₁-C₄-Halogenalkylgruppe bedeuten und R² darüber hinaus auch für eine Trichlormethoxygruppe steht, mit Antimontrifluorid, gegebenenfalls in Gegenwart katalytischer Mengen eines Antimon(V)-salzes oder mit Fluorwasserstoff als Halogenaustauschmittel behandelt.

3. Verfahren nach Anspruch 2, dadurch gekennzeichnet, daß man parallel zur Ausbildung der Trifluor- bzw. Chlordifluormethoxygruppe aus dem Ausgangsstoff II auch einen der Reste R² oder R³ mit der Bedeutung Trichlormethyl gleichzeitig zu dem entsprechenden Trifluormethylrest umsetzt.

4. Verfahren nach den Ansprüchen 2 und 3, dadurch gekennzeichnet, daß man als Halogenaustauschmittel Antimontrifluorid, gegebenenfalls in Gegenwart katalytischer Mengen eines Antimon(V)-salzes, verwendet und die Umsetzung bei einer Temperatur von 90 bis 170°C durchführt.

5. 2,4-Difluor-6-trifluormethoxy-pyrimidin.

6. 6-Chlordifluormethoxy-2,4-difluor-pyrimidin.

7. 2-Fluor-4-trifluormethoxy-6-trifluormethyl-pyrimidin.

8. 2,4-Dichlor-6-trifluormethoxy-pyrimidin.

9. Substituierte Trichlormethoxy-pyrimidine der allgemeinen Formel II 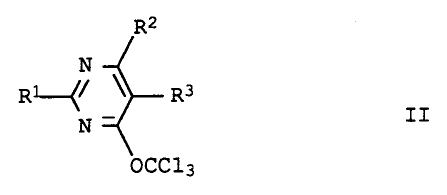 in der R¹ Fluor oder Chlor bedeutet und R² und R³ unabhängig voneinander Wasserstoff, ein Halogenatom oder eine C₁-C₄-Halogenalkylgruppe bedeuten und R² darüber hinaus auch für einen Trichlormethoxyrest steht.

10. Verfahren zur Herstellung von Trichlormethoxypyrimiden der allgemeinen Formel II nach Anspruch 9, dadurch gekennzeichnet, daß man Methoxy-pyrimidinverbindungen der Formel III, 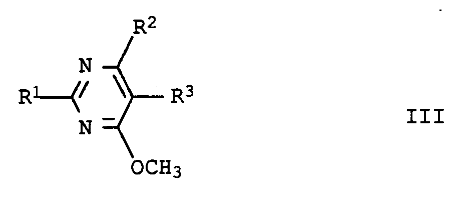 in der R¹ Fluor oder Chlor bedeutet und R² und R³ unabhängig voneinander Wasserstoff, ein Halogenatom oder eine C₁-C₄-Halogenalkylgruppe bedeuten und R² darüber hinaus auch für eine Methoxygruppe steht, mit einem Chlorierungsmittel behandelt.

11. Verfahren nach Anspruch 10, dadurch gekennzeichnet, daß man die Chlorierung mit Chlorgas bei einer Temperatur von 60 bis 180°C durchführt.

12. Verfahren nach den Ansprüchen 10 und 11, dadurch gekennzeichnet, daß man die Chlorierung in Gegenwart von Radikalstartern durchführt.

13. 2,4-Dichlor-6-trichlormethoxy-pyrimidin.

14. 2,4-Difluor-6-trichlormethoxy-pyrimidin.

15. 2-Chlor-4-trichlormethoxy-6-trichlormethylpyrimidin.

## Claims

1. A substituted trifluoro- or chlorodifluoromethoxypyrimidine of the formula I 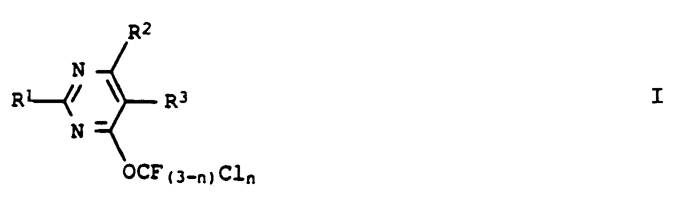 where R¹ is fluorine or chlorine and R² and R³ are each, independently of one another, hydrogen, halogen or C₁-C₄-haloalkyl, and R² is also trifluoromethoxy or chlorodifluoromethoxy, and n is 0 or 1.

2. A process for preparing a trifluoro- or chlorodifluoromethoxypyrimidine of the formula I as claimed in claim 1, which comprises carrying out a halogen replacement on a trichloromethoxypyrimidine of the formula II 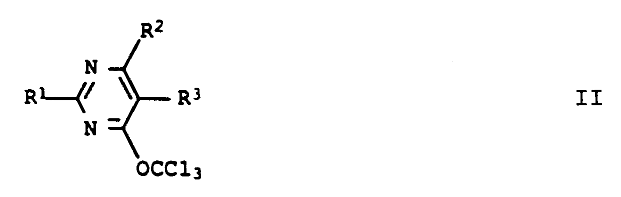 where R¹ is fluorine or chlorine and R² and R³ are each, independently of one another, hydrogen, halogen or C₁-C₄-haloalkyl, and R² is also trichloromethoxy, with antimony trifluoride in the presence or absence of a catalytic amount of an antimony(V) salt, or with hydrogen fluoride.

3. A process as claimed in claim 2, wherein the formation of the trifluoromethoxy or chlorodifluoromethoxy group takes place in parallel with the conversion of one of the radicals R² or R³ meaning trichloromethyl in the starting material II into a trifluoromethyl radical.

4. A process as claimed in claims 2 and 3, wherein the halogen replacement is carried out with antimony trifluoride in the presence or absence of a catalytic amount of an antimony(V) salt, and the reaction is carried out at from 90 to 170°C.

5. 2,4-Difluoro-6-trifluoromethoxypyrimidine.

6. 6-Chlorodifluoromethoxy-2,4-difluoropyrimidine.

7. 2-Fluoro-4-trifluoromethoxy-6-trifluoromethyl-pyrimidine.

8. 2,4-Dichloro-6-trifluoromethoxypyrimidine.

9. A substituted trichloromethoxypyrimidine of the formula II 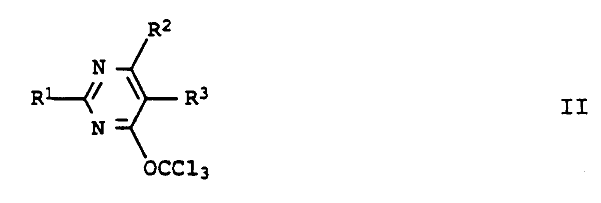 where R¹ is fluorine or chlorine and R² and R³ are each, independently of one another, hydrogen, halogen or C₁-C₄-haloalkyl, and R² is also trichloromethoxy.

10. A process for preparing a trichloromethoxypyrimidine of the formula II as claimed in claim 9, which comprises chlorinating a methoxypyrimidine of the formula III 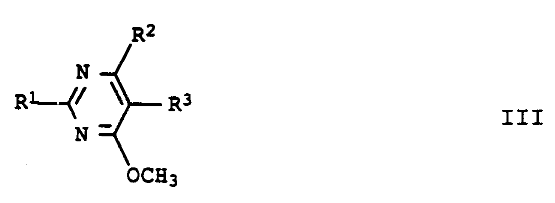 where R¹ is fluorine or chlorine and R² and R³ are each, independently of one another, hydrogen, halogen or C₁-C₄-haloalkyl, and R² is also methoxy.

11. A process as claimed in claim 10, wherein the chlorination is carried out with chlorine gas at from 60 to 180°C.

12. A process as claimed in claims 10 and 11, wherein the chlorination is carried out in the presence of a radical initiator.

13. 2,4-Dichloro-6-trichloromethoxypyrimidine.

14. 2,4-Difluoro-6-trichloromethoxypyrimidine.

15. 2-Chloro-4-trichloromethoxy-6-trichloromethyl-pyrimidine.

## Revendications

1. Trifluoro- et chloro-difluoro-méthoxy-pyrimidines substituées de formule générale I 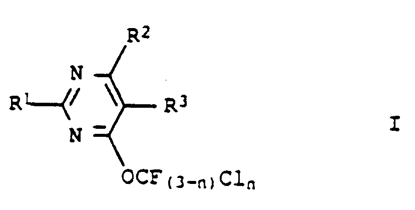 dans laquelle R¹ représente le fluor ou le chlore et R² et R³ représentent chacun, indépendamment l'un de l'autre, l'hydrogène, un atome d'halogène ou un groupe halogénoalkyle en C1-C4, R² pouvant également représenter un groupe trifluoro- ou chlorodifluorométhoxy, et n est égal à 0 ou 1.

2. Procédé de préparation des trifluoro- et chlorodifluoro-méthoxypyrimidines de formule générale I de la revendication 1, caractérisé en ce que l'on traite des trichlorométhoxy-pyrimidines de formule II 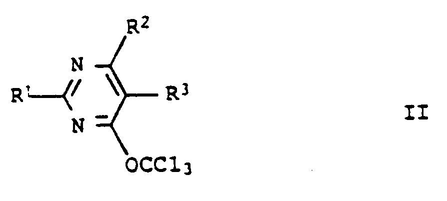 dans laquelle R¹ représente le fluor ou le chlore et R² et R³ représentent chacun, indépendamment l'un de l'autre, l'hydrogène, un atome d'halogène ou un groupe halogénoalkyle en C1-C4, R² pouvant en outre représenter un groupe trichlorométhoxy, par le trifluorure d'antimoine, éventuellement en présence de quantités catalytiques d'un sel d'antimoine-V, ou par le fluorure d'hydrogène, en tant qu'agent échangeur d'halogène.

3. Procédé selon la revendication 2, caractérisé en ce que, parallèlement à la formation du groupe trifluoro- ou chlorodifluorométhoxy à partir du composé de départ II, l'un des groupes R² ou R³, consistant en un groupe trichlorométhyle, est converti simultanément en le groupe correspondant trifluorométhyle.

4. Procédé selon les revendications 2 et 3, caractérisé en ce que l'on utilise, en tant qu'agent échangeur d'halogène, le trifluorure d'antimoine, éventuellement en présence de quantités catalytiques d'un sel d'antimoine-V, et on effectue la réaction à une température de 90 à 170°C.

5. La 2,4-difluoro-6-trifluorométhoxy-pyrimidine.

6. La 6-chlorodifluorométhoxy-2,4-difluoro-pyrimidine.

7. La 2-fluoro-4-trifluorométhoxy-6-trifluorométhyl-pyrimidine.

8. La 2,4-dichloro-6-trifluorométhoxy-pyrimidine.

9. Trichlorométhoxypyrimidines substituées de formule générale II 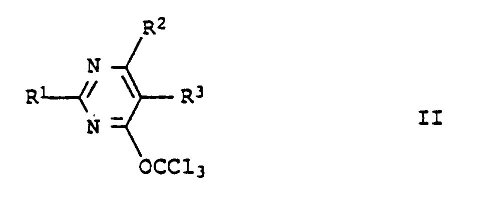 dans laquelle R¹ représente le fluor ou le chlore et R² et R³ représentent chacun, indépendamment l'un de l'autre, l'hydrogène, un atome d'halogène ou un groupe halogénoalkyle en C1-C4, R² pouvant en outre représenter un groupe trichlorométhoxy.

10. Procédé de préparation des trichlorométhoxypyrimidines de formule générale II de la revendication 9, caractérisé en ce que l'on traite par un agent chlorant des méthoxypyrimidines de formule III 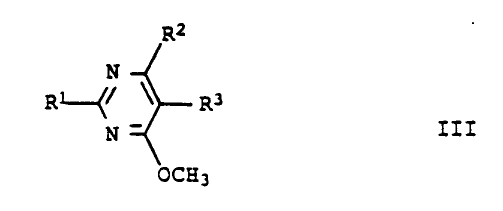 dans laquelle R¹ représente le fluor ou le chlore et R² et R³ représentent chacun, indépendamment l'un de l'autre, l'hydrogène, un atome d'halogène ou un groupe halogénoalkyle en C1-C4, R² pouvant en outre représenter un groupe méthoxy.

11. Procédé selon la revendication 10, caractérisé en ce que l'on chlore à l'aide du chlore gazeux à une température de 60 à 180°C.

12. Procédé selon les revendications 10 et 11, caractérisé en ce que l'on procède à la chloruration en présence d'inducteurs radicalaires.

13. La 2,4-dichloro-6-trichlorométhoxy-pyrimidine.

14. La 2,4-difluoro-6-trichlorométhoxy-pyrimidine.

15. La 2-chloro-4-trichlorométhoxy-6-trichlorométhyl-pyrimidine.
